# EUROPÄISCHE PATENTANMELDUNG

(11) **EP 0 556 705 A1**
(43) Veröffentlichungstag der Anmeldung: **25.08.1993**
(21) Anmeldenummer: 93102074.7
(22) Anmeldetag: 10.02.1993
(51) Int. Cl.: A61B 17/39

(54) **Hochfrequenzschirurgiegerät**

(30) Priorität: 20.02.1992 DE 4205213
(71) Anmelder: DELMA ELEKTRO-UND MEDIZINISCHE APPARATEBAU GESELLSCHAFT mbH, D-78532 Tuttlingen (DE)
(72) Erfinder: Dornhof, Konstantin, W-7208 Spaichingen (DE)
(74) Vertreter: Morgan, James Garnet

(57) **Zusammenfassung**

Ein Hochfrequenzchirurgiegerät weist einen zwei an ein Gewebe anlegbare Elektroden (11, 12) beaufschlagenden, regelbaren Hochfrequenzgenerator (13) auf, der eine bezüglich seiner Ausgangsleistung steuerbare Stromversorgung (14) und einen von dieser gespeisten, eine Leistungsendstufe umfassenden Oszillator (15) besitzt und in Abhängigkeit von der Einstellung der Stromversorgung (14) und dem Gewebewiderstand (R) zwischen den Elektroden (11, 12) eine vorbestimmte Ausgangsleistung abgibt. In einem Digitalspeicher (19) ist eine Anzahl vorgegebener Kurven, die für einen bestimmten funktionellen Zusammenhang zwischen der Ausgangsleistung des Hochfrequenzgenerators (13) und dem Gewebewiderstand (R) repräsentativ sind und die Kennlinien des Hochfrequenzgenerators (13) berücksichtigen, in digitaler Form abgelegt. An eine mit dem Digitalspeicher (19) verbundene Steuereinheit (21) ist eine dem aktuellen Gewebewiderstand (R) entsprechende und eine der ausgewählten Betriebsart entsprechende Steueradresse angelegt.

## Beschreibung

Die Erfindung betrifft ein Hochfrequenzchirurgiegerät nach dem Oberbegriff des Patentanspruchs 1.

Derartige Hochfrequenzchirurgiegeräte dienen zum Schneiden und Koagulieren von Gewebe, wozu jeweils Strom- und Spannungsverläufe über dem Gewebewiderstand gewährleistet werden müssen, die so angepaßt sind, daß bei unterschiedlichen Widerständen und Ausgangsleistungen die jeweilige Schneid- oder Koagulationsfunktion erfüllt wird. Bei bekannten Geräten wählt man zunächst mittels einer Wahltastatur beispielsweise zwischen den Betriebsarten Schneiden, Koagulieren bzw. Bipol-Koagulation. Für jede Betriebsart kann dann noch eine gewünschte Ausgangsleistung vorgewählt werden, die bei der Betriebsart "Schneiden" z.B. zwischen 10 und 400 W, bei der Betriebsart "Koagulation" zwischen 10 und 300 W und bei der Betriebsart "Bipol-Koagulation" zwischen 10 und 50 W liegen kann.

Ein Problem bei den bekannten Hochfrequenzchirurgiegeräten besteht also darin, daß für die unterschiedlichen Betriebsarten die Stromversorgung des Hochfrequenzgenerators ganz unterschiedlich angesteuert werden muß, damit für jeden zwischen den Elektroden vorhandenen Gewebewiderstand wenigstens in etwa die gewünschte Ausgangsleistung bereitgestellt wird. Bei den bekannten Geräten werden die gewünschten Leistungs-Widerstands-Kennlinien der vorgewählten Betriebsarten nur unvollkommen eingehalten, so daß eine genaue Anpassung des Hochfrequenzgenerators an ein spezielles Gewebe-Behandlungsproblem nicht möglich ist.

Das Ziel der vorliegenden Erfindung besteht darin, ein Hochfrequenzchirurgiegerät der eingangs genannten Gattung zu schaffen, bei dem für eine Fülle unterschiedlicher Betriebsarten gewünschte Leistungs-Widerstands-Kennlinien vorgegeben werden können und beim späteren Betrieb nach entsprechender Auswahl der Betriebsart durch die Bedienungsperson exakt eingehalten werden.

Zur Lösung dieser Aufgabe sind die Merkmale des kennzeichnenden Teils des Patentanspruchs 1 vorgesehen.

Der Erfindungsgedanke ist also darin zu sehen, daß man in einem Digitalspeicher, z.B. einem Nur-Lese-Speicher (ROM) die für die verschiedenen Betriebsarten gewünschten Ausgangsleistungs-Gewebewiderstands-Kennlinien bzw. -Kurvenverläufe ablegt und durch die Wähleinrichtung und die Messung des aktuellen Gewebewiderstandes den Hochfrequenzgenerator derart zwangssteuert, daß er in Abhängigkeit vom aktuellen Gewebewiderstand exakt diejenige Ausgangsleistung abgibt, die für diesen Gewebewiderstand in der im Digitalspeicher angewählten Kennlinie vorgeschrieben ist.

Da in einem Digitalspeicher eine große Vielzahl unterschiedlicher Kurvenverläufe ablegbar ist, kann somit eine große Fülle von unterschiedlichen Betriebsarten beim erfindungsgemäßen Hochfrequenzchirurgiegerät nicht nur vorgegeben, sondern auch exakt eingehalten werden.

Die Wähleinrichtung kann erfindungsgemäß Einstellmittel für verschiedene Betriebsarten aufweisen, wobei für jede Betriebsart auch noch unterschiedliche Durchschnittsleistungsstufen ausgewählt werden können. In dem Digitalspeicher sind also für jede Betriebsart Leistungs-Gewebewiderstands-Kurvenscharen abgelegt, die ebenso wie die Betriebsart selbst mittels der Wähleinrichtung ausgewählt werden können.

Vorteilhafte Weiterbildungen der Erfindung sind durch die Unteransprüche gekennzeichnet.

Aufgrund der Ausbildung nach Anspruch 11 können Hochfrequenzchirurgiegeräte auch noch nach der Fertigstellung mit einem geeigneten Digitalspeicher ausgestattet werden; auch ist so jederzeit eine Auswechslung und damit eine Anpassung an andere Erfordernisse möglich.

Der Gewebewiderstand wird am einfachsten durch Messung von Ausgangsspannung und Ausgangsstrom des Hochfrequenzgenerators ermittelt. Ausgangsstrom und Ausgangsspannung können entweder unmittelbar oder mittelbar durch Sensoren ermittelt werden.

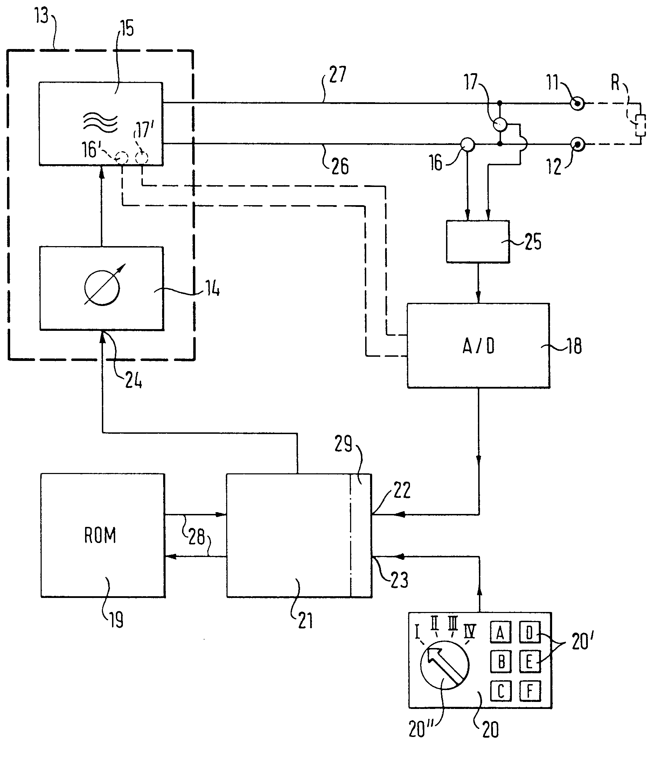

Die Erfindung wird im folgenden beispielsweise anhand der Zeichnung beschrieben, deren einzige Figur ein schematisches Blockschaltbild eines Hochfrequenzchirurgiegerätes zeigt.

Nach der Zeichnung kann ein Hochfrequenzgenerator 13 einen Oszillator 15, der auch eine Leistungsendstufe und gegebenenfalls Vorverstärker enthält, sowie eine regelbare Stromversorgung 14 mit einem Steuereingang 24 umfassen. Der Oszillator 15 speist über Hochfrequenz-Ausgangsleitungen 26, 27 zwei Elektroden 11, 12, die an das Gewebe eines Patienten, z.B. als Schneid- oder Koagulationselektroden bzw. aktive und Neutralelektrode angelegt werden können. Der zwischen den Elektroden 11, 12 wirksame Gewebewiderstand R ist gestrichelt angedeutet.

An den Ausgangsleitungen 26, 27 sind ein Stromsensor 16 und ein Spannungssensor 17 angeordnet, die an einen Analog-Rechenschaltung 25 angelegt sind, der aus den gemessenen Strom- und Spannungswerten den Gewebewiderstand R ermittelt und ein entsprechendes Signal einem Analog-Digital-Wandler 18 zuführt. Der Analog-Digital-Wandler 18 liefert an den ersten Eingang 22 einer eine Adreß-Dekodiereinheit 29 enthaltenden Steuereinheit 21 eine Steueradresse, die für den Gewebewiderstand R repräsentativ ist. An einen zweiten Kurvenauswahl-Eingang 23 der Steuereinheit 21 ist eine von einer Wähleinrichtung 20 abgeleitete Kurvenauswahl-Steueradresse angelegt, die beispielsweise mittels einer schematisch angedeuteten Tastatur 20' mit Tasten A bis E und mittels eines Stellers 20'' mit Stellungen I bis IV ausgewählt werden kann. Mit der Tastatur 20' können z.B. verschiedene Betriebsarten wie Koagulation, Schneiden, Bipolkoagulation, ausgewählt werden, während der Steller die Einstellung verschiedener Durchschnittsleistungspegel bei diesen Betriebsarten ermöglicht. Die Adreß-Dekodiereinheit 29 erkennt und dekodiert die Steueradressen.

Die Steuereinheit 21 durch Dialogleitungen 28 mit einem Digitalspeicher 19 verbunden, der z.B. als Nur-Lese-Speicher (ROM) ausgebildet ist. Der Ausgang der Steuereinheit 21 liegt am Steuereingang 24 der Stromversorgung 14 des Oszillators 15 an.

Die Arbeitsweise des beschriebenen Hochfrequenzchirurgiegerätes ist wie folgt:
Zunächst werden in den Digitalspeicher 19 die gewünschten Ausgangsleistungs-Gewebewiderstands-Kennlinien eingegeben und dort in digitaler Form abgelegt. Dabei können sämtliche gewünschten Kennlinien in jeder digitalen Abstufung vorgegeben werden, sofern die Stromversorgung 14 und der Oszillator 15 für die in den Kennlinien vorkommenden Ausgangsleistungen dimensioniert sind. Ein derart programmierter Digitalspeicher 19 wird dann in das Gerät entweder bei der Herstellung eingebaut oder vom Benutzer je nach seinen Bedürfnissen als Modul oder Speicherkarte in das bereits soweit fertiggestellte Gerät eingesteckt.

Soll das Gerät dann auf eine bestimmte Kennlinie eingestellt werden, so drückt der Benutzer an der Wähleinrichtung 20 für die ausgewählte Betriebsart die zugeordnete Taste der Tastatur 20' und stellt außerdem mit dem Steller 20'' die gewünschte Basisleistung ein. Über den Eingang 23 wird dadurch der Steuereinheit 21 eine entsprechende Steueradresse zugeführt.

Die Strom-Spannungs-Sensoren 16, 17 messen bevorzugt an den Ausgangsleitungen 26, 27 den Ausgangsstrom und die Ausgangsspannung des Hochfrequenzgenerators 13 und geben die gemessenen Werte an die Analog-Rechenschaltung 25 weiter, die daraus einen für den Gewebewiderstand R repräsentativen Wert ermittelt und an einen Analog-Digital-Wandler 18 abgibt. Dieser liefert dann eine digitale Steueradresse an den ersten Eingang 22 der Steuereinheit 21. Diese Steueradresse ist repräsentativ für den zwischen den Elektroden 11, 12 vorhandenen Gewebewiderstand R.

In Abhängigkeit von den bei 22, 23 angelegten Steueradressen ruft die Steuereinheit 21 aus dem digitalen Speicher 19 den auf der ausgewählten Kennlinie für den aktuellen Gewebewiderstand R vorgesehenen Leistungswert ab, mittels dessen über den Steuereingang 24 die Stromversorgung 14 auf einen solchen Wert eingeregelt wird, daß am Ausgang des Hochfrequenzgenerators 13 gerade diejenige Ausgangsleistung zur Verfügung gestellt wird, die die ausgewählte Kennlinie für den aktuellen Gewebewiderstand R vorgibt. Auf diese Weise wird der Hochfrequenzgenerator 13 zwangsgesteuert, indem einem sich am Gewebe einstellenden Widerstand R zwingend die der ausgewählten Kennlinie entsprechende Ausgangsleistung zugeordnet wird. Auf diese Weise kann jede gewünschte funktionelle Beziehung zwischen der Ausgangsleistung des Hochfrequenzgenerators 13 und dem Gewebewiderstand R durch entsprechende Eingabe in den digitalen Speicher 19 vorgegeben werden.

Während die Messung von Strom und Spannung durch an den Ausgangsleitungen 26, 27 vorgesehene Sensoren 16, 17 bevorzugt ist, ist es auch möglich, für Ausgangsstrom und Ausgangsspannung repräsentative Werte durch Anordnung von Sensoren 16', 17' an geeigneter Stelle im Hochfrequenzgenerator 13 zu ermitteln und entweder direkt (gestrichelte Darstellung in der Zeichnung) oder über die Analog-Rechenschaltung 25 zum Analog-Digital-Wandler 18 zu schicken. Insbesondere können die beiden Sensoren 16', 17' zwischen der steuerbaren Stromversorgung 14 und dem Oszillator 15 angeordnet sein.

Die Sensoren 16, 17 können auch unmittelbar an den Analog-Digital-Wandler 18 angeschlossen werden.

Weiter ist es möglich, daß die von den Sensoren 16, 17 bzw. 16', 17' gewonnenen Signale einem digitalen Prozessor zugeführt, dort verarbeitet und erst dann an die Steuereinheit 21 weitergeleitet werden.

## Patentansprüche

1. Hochfrequenzchirurgiegerät mit einem zwei an ein Gewebe anlegbare Elektroden (11, 12) beaufschlagenden, leistungsregelbaren Hochfrequenzgenerator (13), der ein bezüglich seiner Ausgangsleistung steuerbare Stromversorgung (14) und einen von dieser gespeisten, eine Leistungsendstufe umfassenden Oszillator (15) aufweist und in Abhängigkeit von der Einstellung der Stromversorgung und dem Gewebewiderstand (R) zwischen den Elektroden (11, 12) eine vorbestimmte Ausgangsleistung abgibt,
dadurch gekennzeichnet,
daß ein Digitalspeicher (19) vorgesehen ist, in dem eine Anzahl vorgegebener Kurven, die für einen bestimmten funktionellen Zusammenhang zwischen der Ausgangsleistung des Hochfrequenzgenerators (13) und dem Gewebewiderstand (R) repräsentativ sind und die Kennlinien des Hochfrequenzgenerators (13) berücksichtigen, in digitaler Form abgelegt ist und welcher mit einer Steuereinheit (21) verbunden ist, an die ein für den aktuellen Gewebewiderstand (R) repräsentatives erstes Eingangssignal oder -Signalpaar und von einer Kurvenwähleinrichtung (20) ein zweites Kurvenauswahl-Eingangssignal angelegt ist und welche in Abhängigkeit von den Eingangssignalen aus dem Digitalspeicher (19) den dem aktuellen Gewebewiderstand (R) bei der ausgewählten Kurve entsprechenden Leistungswert abruft und aufgrund dieses Leistungswertes die Stromversorgung (14) derart ansteuert, daß der Hochfrequenzgenerator (13) an die Elektroden (11, 12) die bei der ausgewählten Kurve für den aktuellen Gewebewiderstand (R) vorgegebene Leistung abgibt.

2. Hochfrequenzchirurgiegerät nach Anspruch 1, dadurch gekennzeichnet, daß der aktuelle Gewebewiderstand R) durch direkte oder indirekte Messung des Ausgangsstromes und der Ausgangsspannung des Hochfrequenzgenerators (13) bestimmt wird, und zwar vorzugsweise durch auf Ausgangsstrom bzw. Ausgangsspannung ansprechende Sensoren (16,17; 16'17').

3. Hochfrequenzchirurgiegerät nach Anspruch 2, dadurch gekennzeichnet, daß die Sensoren (16, 17; 16', 17') Analogsignale abgeben und daß zwischen die Sensoren (16, 17; 16', 17') und die Steuereinheit (21) sowie den Digitalspeicher (19) ein Analog-Digital-Wandler (18) geschaltet ist, der die Analogsignale in eine für den Gewebewiderstand (R) repräsentative Steueradresse für die Steuereinheit (21) und den Digitalspeicher (19) umwandelt.

4. Hochfrequenzchirurgiegerät nach Anspruch 3, dadurch gekennzeichnet, daß die Ausgangssignale der Sensoren (16, 17) über eine Analog-Rechenschaltung (25) an den Analog-Digital-Wandler (23) angelegt sind.

5. Hochfrequenzchirurgiegerät nach einem der vorhergehenden Ansprüche, dadurch gekennzeichnet, daß die Sensoren (16', 17') zwischen der Stromversorgung (14) und dem Oszillator (15) angeordnet sind.

6. Hochfrequenzchirurgiegerät nach einem der Ansprüche 1 bis 4, dadurch gekennzeichnet, daß die Sensoren (16, 17) an bzw. in den Hochfrequenzausgangsleitungen (26, 27) angeordnet sind.

7. Hochfrequenzchirurgiegerät nach einem der vorhergehenden Ansprüche, dadurch gekennzeichnet, daß die Steuereinheit (21), der Digitalspeicher (19) und/oder der Analog-Digital-Wandler (18) Bestandteil eines Rechenbausteins, z.B. eines Mikroprozessors, sind.

8. Hochfrequenzchirurgiegerät nach einem der vorhergehenden Ansprüche, dadurch gekennzeichnet, daß die Steuereinheit (21) eine Adreß-Dekodiereinheit (29) aufweist.

9. Hochfrequenzchirurgiegerät nach einem der vorhergehenden Ansprüche, dadurch gekennzeichnet, daß der Digitalspeicher (19) ein Nur-Lese-Speicher (ROM) ist.

10. Hochfrequenzchirurgiegerät nach einem der vorhergehenden Ansprüche, dadurch gekennzeichnet, daß der Inhalt des Digitalspeichers (19) löschbar und erneuerbar ist.

11. Hochfrequenzchirurgiegerät nach einem der vorhergehenden Ansprüche, dadurch gekennzeichnet, daß der Digitalspeicher (19) als auswechselbarer bzw. nachträglich einsetzbarer Modul, Steckkarte od.dgl. ausgebildet ist.
